# EUROPEAN PATENT SPECIFICATION

(11) **EP 1 671 635 B1**
(45) Date of publication and mention of the grant of the patent: **04.01.2012**
(21) Application number: 04792137.4
(22) Date of filing: 07.10.2004
(51) Int. Cl.: A61K 9/14, A61K 31/546, A61K 9/00

(54) **NONCRYSTALLINE ANTIBACTERIAL COMPOSITION CONTAINING CEFDITOREN PIVOXIL**
NICHTKRISTALLINE ANTIBAKTERIELLE ZUSAMMENSETZUNG MIT CEFDITOREN-PIVOXIL
COMPOSITION ANTIBACTERIENNE NON CRISTALLINE CONTENANT DU CEFDITOREN PIVOXIL

(30) Priority: 08.10.2003 JP 2003349167
(43) Date of publication of application: 21.06.2006
(73) Proprietor: Meiji Seika Pharma Co., Ltd., Chuo-ku Tokyo (JP)
(72) Inventor: CHIKASE, Shigeru; c/o Meiji Seika Kaisha, Ltd., Yokohama-shi, Kanagawa; 2228567 (JP); YOKOI, Yukiko; c/o Meiji Seika Kaisha, Ltd., 760,, Kanagawa 2228567 (JP)
(74) Representative: Ahner, Francis
(86) International application number: PCT/JP2004/014840
(87) International publication number: WO 2005/034957

(56) References cited:
- EP-A- 1 555 024
- EP-A1- 1 051 978
- EP-A2- 1 080 721
- WO-A1-00/04896
- WO-A1-96/19239
- WO-A1-02/087588
- JP-A- 7 017 866
- JP-A- 2001 131 071
- JP-A- 2003 026 676
- JP-A- 2004 175 779
- DATABASE EPODOC EUROPEAN PATENT OFFICE, THE HAGUE, NL; XP002435617 & JP 60 188320 A (ASAHI CHEMICAL IND) 25 September 1985 (1985-09-25)
- DATABASE EPODOC EUROPEAN PATENT OFFICE, THE HAGUE, NL; XP002435618 & JP 60 132918 A (ASAHI CHEMICAL IND) 16 July 1985 (1985-07-16)
- DATABASE WPI Week 198533 Derwent Publications Ltd., London, GB; AN 1985-200315 XP002435634 & JP 60 126230 A (ASAHI CHEM IND CO LTD) 5 July 1985 (1985-07-05)
- YOKOI YUKIKO ET AL: "Effects of sugar ester and hydroxypropyl methylcellulose on the physicochemical stability of amorphous cefditoren pivoxil in aqueous suspension" INTERNATIONAL JOURNAL OF PHARMACEUTICS (KIDLINGTON), vol. 290, no. 1-2, 16 February 2005 (2005-02-16), pages 91-99, XP004967394 ISSN: 0378-5173

## Description

### BACKGROUND OF THE INVENTION

The present invention relates to amorphous antibiotic compositions comprising cefditoren pivoxil.

An antibiotic compound cefditoren is a cephem compound represented by formula (A): 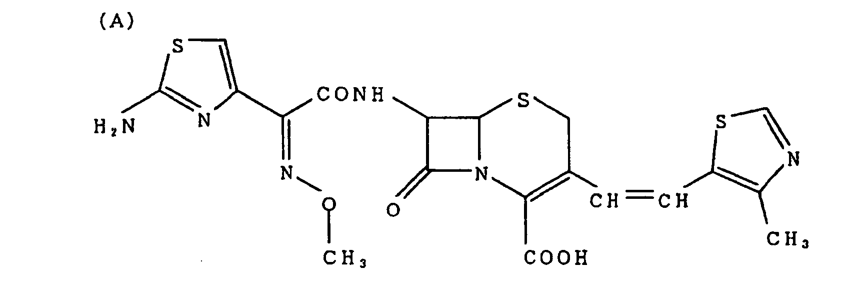 Its chemical name is (+)-(6R,7R)-7-[(Z)-2-(2-aminothiazol-4-yl)-2-methoxyiminoace tamido]-3-[(Z)-2-(4-methylthiazol-5-yl)ethenyl]-8-oxo-5-thia -1-azabicyclo[4.2.0]oct-2-ene-2-carboxylic acid. This compound is described in Japanese Patent Publication No.64503/1991 under the chemical name of 7-[2-methoxyimino-2-(2-aminothiazol-4-yl)acetamido]-3-[2-(4-methylthiazol-5-yl)vinyl]-3-cephem-4-carboxylic acid (syn-isomer, cis-isomer).

A pivaloyloxymethyl ester of cefditoren, in which a carboxylic acid group on position 2 of the cephem compound is esterified with a pivaloyloxymethyl group for the purpose of improving its absorbability through the digestive tracts upon oral administration (hereinafter referred to as "oral absorbability"), is called cefditoren pivoxil. This prodrug compound is represented by formula (B): 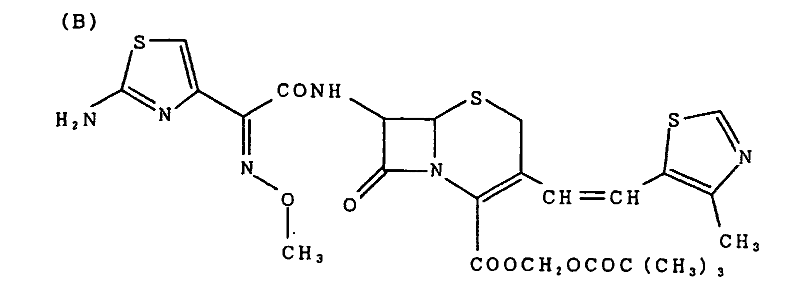 and its chemical name is (-)-(6R,7R)-7-[(Z)-2-(2-aminothiazol-4-yl)-2-methoxyiminoace tamido]-3-[(Z)-2-(4-methylthiazol-5-yl)ethenyl]-8-oxo-5-thia -1-azabicyclo[4.2.0]oct-2-ene-2-carboxylic acid 2,2-dimethylpropionyloxymethyl ester. This ester compound is generally considered to exhibit high oral absorbability as compared to the original acid-form drug. However, the esterification of cefditoren has not necessarily resulted in enhancement or improvement of the oral absorbability to the satisfactory level.

Japanese Patent No. 3413406 discloses a composition comprising a crystallographically stable, amorphous cephalosporin and a process for the preparation thereof, indicating that the oral absorbability can be improved by amorphousizing the cephalosporin. Japanese Patent Laid-Open Publication No. 131071/2001 discloses a process for the preparation of amorphous cefditorenpivoxil, in which the oral absorbability can be improved by amorphousizing cefditoren pivoxil. Further, WO 02/87588 discloses a process for producing an amorphous composition, in which an organic polymer is mixed with cefditoren pivoxil crystals and the obtained mixture is ground.

On the other hand, as a means to improve oral absorbability of a poorly soluble drug, a solid composition which is obtained by amorphousizing the poorly soluble drug in the presence of a polymer base and a nonionic surfactant is disclosed in WO 96/19239. It is disclosed that when the abovementioned composition is dispersed in a liquid, microgranules having a diameter of less than 1 µm are formed and thus the drug maintains its amorphous state. However, such amorphousness-maintaining effect was not observed for combinations of drugs and nonionic surfactants. Further, since 0.5 to 20 parts by weight of polymer base and 0.1 to 3 parts by weight of nonionic surfactant were added to the drug in the disclosed solid composition, the resulting pharmaceutical preparation such as an antibiotic drug with 100 mg potency tablet became bulky and thus pharmaceutical tablets or granules became bulky in the same way as mentioned above, which made oral administration difficult.

Furthermore, as a pharmaceutical preparation to improve the oral absorbability of cefditoren pivoxil, a pharmaceutical preparation in which cyclodextrin or hydroxypropyl cellulose that is a water-soluble polymer cellulose derivative is added to cefditoren pivoxil has been proposed (Japanese Patent Publication No. 78234/1994 and Japanese Patent Laid-Open Publication No. 17866/1995). However, the addition of cyclodextrin to cefditoren pivoxil extremely intensified the bitterness derived from cefditoren pivoxil and pharmaceutical tablets or granules obtained with the addition of hydroxypropyl cellulose became bulky, which made oral administration difficult.

In order to solve these problems, a pharmaceutical preparation in which a water-soluble caseinate is added to cefditoren pivoxil has recently been proposed (Japanese Patent No. 2831135) . However, this preparation could not be administered to a patient suffering from a milk allergy since casein is a protein derived from milk.

Thus, a pharmaceutical preparation wherein cefditoren pivoxil can be safely administered to a patient with oral absorbability sufficient enough to surely exert its expected pharmaceutical effect has been needed. Further, in dry syrup and a liquid preparation such as syrup which are orally administered in an appropriately divided dose by dissolving or suspending a drug in a medium, the drug has to be maintained in its dissolved state for a long period of time.

### DISCLOSURE OF THE INVENTION

Since amorphous cefditoren pivoxil is apt to change into a crystalline state in a solution as shown in the prior art, a composition comprising amorphous cefditoren pivoxil still needs to be improved.

Accordingly, an obj ect of the present invention is to provide a cefditoren pivoxil composition which can maintain highly orally absorbable amorphous cefditoren pivoxil in a suspension for a long period of time, thereby being useful as a material for a pharmaceutical preparation.

The present inventors have now found that crystallization of amorphous cefditoren pivoxil in a suspension was inhibited by suspending a solid dispersion comprising cefditoren pivoxil and a sugar ester fatty acid in a medium.

According to the present invention, there is provided a solid dispersion composition (referred to as "composition according to the present invention" hereinafter) comprising at least 0.1 mg of a sugar ester fatty acid on the basis of an amount equivalent to 100 mg potency of cefditoren pivoxil.

The solid dispersion composition according to the present invention can maintain the amorphous state of cefditoren pivoxil in a suspension for a long period of time. Therefore, the solid dispersion composition according to the present invention is useful as a material for a pharmaceutical preparation of cefditoren pivoxil, in particular it opens the way for a pharmaceutical preparation that can be administered by suspending it upon administration.

### BEST MODE OF CARRYING OUT THE INVENTION

Cefditoren pivoxil to be used as a material for the solid dispersion composition according to the present invention can be commercially available products or may be produced according to a known method. Cefditoren pivoxil can be produced according to the method described in Japanese Patent Publication No. 64503/1991. Further, amorphous cefditoren pivoxil described in Japanese Patent No. 3413406 and crystalline cefditoren pivoxil described in Japanese Patent No. 3403206 can be used.

A sugar ester fatty acid added to the solid dispersion composition according to the present invention can be used by selecting from commercially available products.

The sugar ester fatty acid can be, not particularly limited to, any ester which is pharmaceutically acceptable and extends the amorphousness-maintaining period for amorphous cefditoren pivoxil. The sugar ester having a high HLB value is preferred and, for example, one with an HLB value of more than 10, preferably 11 to 20 , can be used. The HLB value can be calculated in accordance with "Standard Methods for Analysis of Fats and Oil" (1971) edited by Japan Oil Chemist' s Society. The sugar ester fatty acid can be used singly or as a mixture of two or more kinds thereof, if necessary.

The amount of the sugar ester fatty acid to be added can be at least 0.1 mg, preferably at least 5 mg, on the basis of an amount equivalent to 100 mg potency of cefditoren pivoxil.

Since the solid dispersion composition according to the present invention is mainly used as a material for a pharmaceutical preparation, an upper limit of the solid dispersion composition to be added is understood by those skilled in the art from a pharmaceutical viewpoint and can be referred to in "Japanese Pharmaceutical Excipients Dictionary 2000" (edited by the Japan Pharmaceutical Excipients Council), if necessary. For example, since the maximum dose for oral administration of a sugar ester fatty acid is 600 mg/day, the upper limit of the amount to be added is 200 mg per dose when administered at 100 mg potency three times a day. However, the upper limit of the amount of the sugar ester fatty acid to be added is preferably 100 mg, more preferably 50 mg, since the resulting formulated preparation becomes bulky when more than 100 mg are added, which makes administration difficult.

The amount of the sugar ester fatty acid to be added can be 0.1 to 200 mg, preferably 5 to 100 mg, more preferably 5 to 50 mg, on the basis of an amount equivalent to 100 mg potency of cefditoren pivoxil.

Preferably, the solid dispersion composition according to the present invention can further contain a pharmaceutically acceptable water-soluble polymer. The amorphousness-maintaining period for cefditoren pivoxil can be markedly extended by adding a pharmaceutically acceptable water-soluble polymer to cefditoren pivoxil together with a sugar ester fatty acid.

The pharmaceutically acceptable water-soluble polymer to be added to the solid dispersion composition according to the present invention can be used by selecting from commercially available products.

The water-soluble polymer can be, not particularly limited to, any polymer which does not inhibit the extension of the amorphousness-maintaining period for cefditoren pivoxil or further extends the amorphousness-maintaining period. For example, hydroxypropylmethyl cellulose (HPMC), methylcellulose (MC), hydroxyethyl cellulose (HEC), polyvinylpyrrolidone (PVP), and hydroxypropyl cellulose (HPC), preferably, HPMC, MC, and HEC, can be used. The water-soluble polymer can be used singly or as a mixture of two or more kinds thereof, if necessary.

The amount of the water-soluble polymer to be added to cefditoren pivoxil can be at least 1 mg on the basis of an amount equivalent to 100 mg potency of cefditoren pivoxil. Since the solid dispersion composition according to the present invention is mainly used as a material for a pharmaceutical preparation, an upper limit of the water-soluble polymer to be added is understood by those skilled in the art from a pharmaceutical viewpoint. For example, the upper limit of the amount of the water-soluble polymer to be added is 100 mg, more preferably 50 mg, since the resulting formulated preparation becomes bulky when more than 100 mg of the water-soluble polymer are added, which makes administration difficult.

The amount of the water-soluble polymer to be added can be 1 to 100 mg, preferably 1 to 50 mg, more preferably 40 to 50 mg, on the basis of an amount equivalent to 100 mg potency of cefditoren pivoxil.

Preferred examples of the solid dispersion composition according to the present invention include one containing 0.1 to 200 mg of the sugar ester fatty acid and 1 to 100 mg of the water-soluble polymer, on the basis of an amount equivalent to 100 mg potency of cefditoren pivoxil, and one containing 5 to 100mgof the sugar ester fatty acid and 1 to 50 mg of the water-soluble polymer, on the basis of an amount equivalent to 100 mg potency of cefditoren pivoxil.

The pharmaceutical solid dispersion composition according to the present invention is produced as a solid dispersion of cefditoren pivoxil, a sugar ester fatty acid and, optionally, a pharmaceutically acceptable water-soluble polymer and/or one or more pharmaceutically acceptable additives.

The term "solid dispersion composition" in the present invention means a solid composition which is produced by the steps of dissolving crystalline or amorphous cefditoren pivoxil as a material for an active ingredient and other ingredients including a sugar ester fatty acid in a solvent and then removing the solvent by distillation, drying, filtration and the like, and is characterized in that the active ingredient and other ingredients including the sugar ester fatty acid are mixed in a molecular state therein. The solid dispersion composition can be produced by methods generally used, including a solventprecipitationmethod, a spray drying method, a freeze drying method, a vacuum drying method, and a kneading method. A spray drying method and a vacuum-drying method are preferably used, in which dichloromethane, methanol, and ethanol can be used as a solvent.

The solid dispersion composition according to the present invention can be made into various forms of pharmaceutical preparations suitable for oral administration by further adding pharmaceutically acceptable additives and formulating by an ordinary method. Therefore, according to the present invention, there is provided an antibiotic pharmaceutical preparation comprising the solid dispersion composition according to the present invention together with pharmaceutically acceptable additives.

Examples of the pharmaceutical preparations suitable for oral administration include powders, fine granules, granules, tablets, and capsules. Examples of the pharmaceutically acceptable additives include excipients, fillers, binding agents, wetting agents, disintegrants, surfactants, lubricants, dispersing agents, buffering agents, preservatives, solution adjuvants, antiseptics, flavoring agents, analgesic agents, and stabilizers.

Upon formulation, a sugar ester fatty acid and a water-soluble polymer can be added. By adding the sugar ester fatty acid and the water-soluble polymer, the amorphousness-maintaining period for amorphous cefditoren pivoxil can be further extended.

The amount of cefditoren pivoxil in the pharmaceutical preparation according to the present invention varies depending on its dosage form. It can be 5 to 90% by weight, preferably 10 to 80% by weight, of the pharmaceutical preparation. The amount of administration for the treatment and prevention of bacterial infection or the like can be appropriately determined by considering the usage, the age and gender of the patient, the severity of the symptoms and the like. An appropriate dose for an adult can be about 300 to 800 mg per day, which can be administered daily as a single or divided dose.

In a preferred embodiment, an example of thesoliddispersion composition or the pharmaceutical preparation according to the present invention has an amorphousness-maintaining period for amorphous cefditoren pivoxil of at least 3 days when suspended in water at a cefditoren pivoxil concentration of 10 mg/ml.

According to a second embodiment of the present invention, there is provided a liquid composition comprising at least 0.1 mg, preferably at least 5 mg of a sugar ester fatty acid, on the basis of an amount equivalent to 100 mg potency of cefditoren pivoxil. This liquid composition can further contain a pharmaceutically acceptable water-soluble polymer. The amounts and specific kinds of the sugar ester fatty acid and the water-soluble polymer to be contained in the liquid composition can be determined according to those of the solid dispersion composition and the antibiotic pharmaceutical preparation according to the present invention. The liquid composition according to the present invention can be obtained by dissolving or suspending the solid dispersion composition according to the present invention or the antibiotic pharmaceutical preparation according to the present invention in amedium (preferably, water). In the liquid composition according to the present invention, the active ingredient cefditoren pivoxil is maintained in its amorphous state for a long period of time. Accordingly, the liquid composition according to the present invention can be used as a pharmaceutical preparation which can be administered by suspending it as needed upon administration.

### EXAMPLES

The present invention will be further illustrated in detail by the following examples that are not intended to restrict the scope of the present invention.

### Reference Example 1: Solid dispersion without surfactant

An amorphous cefditoren pivoxil composition was obtained by co-precipitating cefditoren pivoxil and a water-soluble polymer 20 in accordance wi th Japanese Patent No. 3413406. Cefditoren pivoxil in this composition was confirmed to be amorphous by the powder X-ray diffraction analysis (data not shown).

### Examples 1, 6, 7, and 8: Solid dispersions containing surfactant

Solid dispersion compositions were obtained by dissolving 25 crystalline cefditoren pivoxil and surfactants in a dichloromethane:methanol (1:1) mixture at the formulation ratios shown in Table 1 and then removing the solvent by distillation. Cefditoren pivoxil in these compositions was confirmed to be amorphous by the powder X-ray diffraction analysis (data not shown) . Crystalline cefditoren pivoxil was prepared in accordance with Japanese Patent No. 3403206.

**Table 1**

| | Surfactant | Formulation ratio (drug: surfactant) |
|---|---|---|
| Example 1 | Sugar ester fatty acid | 100 mg potency : 5.0 mg |
| Example 6 | Sugar ester fatty acid | 100 mg potency : 0.01 mg |
| Example 7 | Sugar ester fatty acid | 100 mg potency : 0.1 mg |
| Example 8 | Sugar ester fatty acid | 100 mg potency : 200 mg |

| | | |
|---|---|---|
| Sugar ester fatty acid: DK Ester SS, HLB value = 20, Daiichi Kogyo Seiyaku Co., Ltd. | | |

### Reference Example 2 and Examples 2, 3, 4, and 5: Solid dispersions containing surfactant and water-soluble polymer

Solid dispersion compositions were obtained by dissolving crystalline cefditoren pivoxil, surfactants and water-soluble polymers in a dichloromethane:methanol (1:1) mixture at the formulation ratios shown in Table 2 and then removing the solvent by distillation. Cefditoren pivoxil in these compositions was confirmed to be amorphous by the powder X-ray diffraction analysis (data not shown). Crystalline cefditoren pivoxil was prepared in accordance with Japanese Patent No. 3403206.

**Table 2**

| | surfactant | Polymer | Formulation ratio (drug: surfactant:polymer) |
|---|---|---|---|
| Reference Example 2 | Tween 80 | HPMC | 100 mg potency : 5.0 mg : 40 mg |
| Example 2 | Sugar ester fatty acid | HPMC | 100 mg potency : 0.5 mg : 1 mg |
| Example 3 | Sugar ester fatty acid | HPMC | 100 mg potency : 0.5 mg : 50 mg |
| Example 4 | Sugar ester fatty acid | HPMC | 100 mg potency : 5.0 mg : 1 mg |
| Example 5 | Sugar ester fatty acid | HPMC | 100 mg potency : 5.0 mg : 50 mg |

| | | | |
|---|---|---|---|
| Sugar ester fatty acid: DK Ester SS, HLB Value = 20, Daiichi Kogyo Seiyaku Co., Ltd. HPMC (hydroxypropylmethyl cellulose) : TC-5R, Shin-Etsu Chemical Co., Ltd. Tween 80 (polyoxyethylene sorbitan fatty acid ester) : TO- 10M, Nikko Chemicals Co. , Ltd. | | | |

### Test Example 1: Evaluation of amorphousness-maintaining period

Suspensions of the compositions obtained in Reference Examples 1 and 2 and Examples 1 to 8 were prepared such that the concentration of amorphous cefditoren pivoxil in the suspensions was 10 mg/ml. More specifically, 350 ml of water were added to cefditoren pivoxil compositions equivalent to 3.5 g potency to obtain each of the suspensions. The amorphousness-maintaining period was evaluated for the suspensions thus prepared.

The amorphousness-maintaining period was measured as follows. Specifically, the suspensions were stored at 25°C under airtight conditions and sampled immediately, 1 day, 2 days, 3 days, 4 days, 7 days, and 10 days after the preparation. The sampled suspensions were centrifuged and the resultant residues were dried under reduced pressure and subjected to the powder X-ray diffraction analysis. The results are shown in Table 3.

**Table 3**

| | Surfactant mixed* | Polymer mixes* (HPMC) | Immediately after the preparation | 1D | 2D | 3D | 4D | 7D | 10D | 14D |
|---|---|---|---|---|---|---|---|---|---|---|
| Reference Example 1 | - | - | A | A | C | C | C | C | C | C |
| Reference Example 2 | Tween 80 5.0 mg | 40 mg | A | C | C | C | C | C | C | C |
| Example 1 | sugar ester fatty acid 5.0 mg | - | A | A | A | C | C | C | C | C |
| Example 2 | Sugar ester fatty acid 0.5 mg | 1 mg | A | A | A | C | C | C | C | C |
| Example 3 | Sugar ester fatty acid 0.5 mg | 50 mg | A | A | A | C | C | C | C | C |
| Example 4 | Sugar ester fatty acid 5.0 mg | 1 mg | A | A | A | A | C | C | C | C |
| Example 5 | Sugar ester fatty acid 5.0 mg | 50 mg | A | A | A | A | A | C | C | C |
| Reference Example 6 | Sugar ester fatty acid 0.01 mg | - | A | A | C | C | C | C | C | C |
| Example 7 | Sugar ester fatty acid 0.1 mg | - | A | A | A | C | C | C | C | C |
| Examples 8 | Sugar ester fatty acid 200 mg | - | A | A | A | A | A | C | C | C |

| | | | | | | | | | | |
|---|---|---|---|---|---|---|---|---|---|---|
| C: Crystalline A: Amorphous Tween 80: Polyoxyethylene sorbitan fatty acid ester crystalline * Formulated on the basis of an amount equivalent to 100 mg potency of crystalline cefditoren pivoxil, except for Reference Examples 1. | | | | | | | | | | |

Crystallization of amorphous cefditoren pivoxil was stimulated wi th the addition of surfactants other than sugar ester fatty acids, while the amorphousness-maintaining period was extended with sugar ester fatty acids. The extension of the amorphousness-maintaining period was observed with the addition of only 0.1 mg of sugar ester fatty acids. Furthermore, the further extension of the amorphousness-maintaining period was observed with the further addition of polymers.

### Pharmaceutical Preparation Example 1

A powdered preparation was produced by mixing 130 g of the composition obtained in Example 1 and 260 g of cornstarch.

### Pharmaceutical Preparation Example 2

1000 capsules were produced by admixing 130 g of the composition obtained in Example 4, 260 g of spray-dried lactose, 130 g of croscarmellose sodium, and 3 g of magnesium stearate and then filling capsules with the admixture.

### Pharmaceutical Preparation Example 3

Homogeneous powder was obtained by mixing 130 g of the composition obtained in Example 1, 390 g of cornstarch, and 480 g of D-mannitol. This homogeneous powder was granulated by wet granulation according to a conventional method to produce a granular preparation.

## Claims

1. A solid dispersion composition comprising at least 0.1 mg, preferably at least 5 mg, of a sugar ester fatty acid on the basis of an amount equivalent to 100 mg potency of cefditoren pivoxil.

2. The solid dispersion composition according to claim 1, comprising 0.1 mg to 200 mg, preferably at least 5 to 100 mg, more preferably 5 to 50 mg, of the sugar ester fatty acid.

3. The solid dispersion composition according to claim 1 or 2, which further comprises a pharmaceutically acceptable water-soluble polymer.

4. The solid dispersion composition according to claim 3, which contains at least 1 mg, preferably 1 to 100 mg, more preferably 1 to 50 mg, of the water-soluble polymer on the basis of an amount equivalent to 100 mg potency of cefditoren pivoxil.

5. The solid dispersion composition according to any one of claims 1 to 4 , which contains 0.1 to 200 mg of the sugar ester fatty acid and 1 to 100 mg of the water-soluble polymer on the basis of an amount equivalent to 100 mg potency of cefditoren pivoxil.

6. The solid dispersion composition according to any one of claims 1 to 4, which contains 5 to 100 mg of the sugar ester fatty acid and 1 to 50 mg of the water-soluble polymer on the basis of an amount equivalent to 100 mg potency of cefditoren pivoxil.

7. The solid dispersion composition according to any one of claims 3 to 6, wherein the pharmaceutically acceptable water-soluble polymer is one or more water-soluble polymers selected from the group consisting of hydroxypropylmethyl cellulose, methylcellulose, hydroxyethyl cellulose, polyvinylpyrrolidone, and hydroxypropyl cellulose.

8. The solid dispersion composition according to any one of claims 1 to 7, wherein the amorphousness-maintaining period of cefditoren pivoxil is at least 3 days when suspended in water at a cefditoren pivoxil concentration of 10 mg/ml.

9. An antibiotic pharmaceutical preparation comprising the composition of any one of claims 1 to 8 together with a pharmaceutically acceptable additive.

10. A liquid composition comprising at least 0.1 mg, preferably at least 5 mg, of the sugar ester fatty acid on the basis of an amount equivalent to 100 mg potency of cefditoren pivoxil, which is obtainable by dissolving or suspending a solid dispersion composition of anyone of claims 1 to8orapharmaceutical preparation of claim 9 in a medium.

## Patentansprüche

1. Eine feste Dispersionszusammensetzung, umfassend mindestens 0,1 mg, vorzugsweise mindestens 5 mg, eines Zucker-Fettsäureesters, bezogen auf eine Menge, die zu 100 mg Wirkstoffgehalt von Cefditoren pivoxil äquivalent ist.

2. Die feste Dispersionszusammensetzung nach Anspruch 1, umfassend 0,1 mg bis 200 mg, vorzugsweise mindestens 5 bis 100 mg, stärker bevorzugt 5 bis 50 mg, des Zucker-Fettsäureesters.

3. Die feste Dispersionszusammensetzung nach Anspruch 1 oder 2, welche weiter ein pharmazeutisch verträgliches wasserlösliches Polymer umfasst. "

4. Die feste Dispersionszusammensetzung nach Anspruch 3, welche mindestens 1 mg, vorzugsweise 1 bis 100 mg, stärker bevorzugt 1 bis 50 mg, des wasserlöslichen Polymers, bezogen auf eine Menge, die zu 100 mg Wirkstoffgehalt von Cefditoren pivoxil äquivalent ist, enthält.

5. Die feste Dispersionszusammensetzung nach einem der Ansprüche 1 bis 4, welche 0,1 bis 200 mg des Zucker-Fettsäureesters und 1 bis 100 mg des wasserlöslichen Polymers, bezogen auf eine Menge, die zu 100 mg Wirkstoffgehalt von Cefditoren pivoxil äquivalent ist, enthält.

6. Die feste Dispersionszusammensetzung nach einem der Ansprüche 1 bis 4, welche 5 bis 100 mg des Zucker-Fettsäureesters und 1 bis 50 mg des wasserlöslichen Polymers, bezogen auf eine Menge, die zu 100 mg Wirkstoffgehalt von Cefditoren pivoxil äquivalent ist, enthält.

7. Die feste Dispersionszusammensetzung nach einem der Ansprüche 3 bis 6, wobei das pharmazeutisch verträgliche wasserlösliche Polymer eines oder mehrere wasserlösliche Polymere, ausgewählt aus der Gruppe bestehend aus Hydroxypropylmethylcellulose, Methylcellulose, Hydroxyethylcellulose, Polyvinylpyrrolidon und Hydroxypropylcellulose, ist.

8. Die feste Dispersionszusammensetzung nach einem der Ansprüche 1 bis 7, wobei der Zeitraum, über den Cefditoren pivoxil seine Amorphität beibehält, mindestens 3 Tage beträgt, wenn es in Wasser bei einer Cefditoren pivoxil-Konzentration von 10 mg/ml suspendiert ist.

9. Eine antibiotische pharmazeutische Zubereitung, umfassend die Zusammensetzung nach einem der Ansprüche 1 bis 8 zusammen mit einem pharmazeutisch verträglichen Additiv.

10. Eine flüssige Zusammensetzung, umfassend mindestens 0,1 mg, vorzugsweise mindestens 5 mg, des Zucker-Fettsäureesters, bezogen auf eine Menge, die zu 100 mg Wirkstoffgehalt von Cefditoren pivoxil äquivalent ist, welche erhältlich ist durch Lösen oder Suspendieren einer festen Dispersionszusammensetzung nach einem der Ansprüche 1 bis 8 oder einer pharmazeutischen Zubereitung nach Anspruch 9 in einem Medium.

## Revendications

1. Composition de dispersion solide comprenant au moins 0,1 mg, de préférence au moins 5 mg, d'un acide gras d'ester de sucre sur la base d'une quantité équivalente à une teneur de 100 mg de Cefditoren pivoxil.

2. Composition de dispersion solide selon la revendication 1, comprenant de 0,1 mg à 200 mg, de préférence au moins 5 à 100 mg, plus préférablement de 5 à 50 mg, de l'acide gras d'ester de sucre.

3. Composition de dispersion solide selon la revendication 1 ou 2, qui comprend en outre un polymère soluble dans l'eau pharmaceutiquement acceptable.

4. Composition de dispersion solide seloh la revendication 3, qui contient au moins 1 mg, de préférence de 1 à 100 mg, plus préférablement de 1 à 50 mg, du polymère soluble dans l'eau sur la base d'une quantité équivalente à une teneur de 100 mg de cefditoren pivoxil.

5. Composition de dispersion solide selon l'une quelconque des revendications 1 à 4, qui contient de 0,1 à 200 mg de l'acide gras d'ester de sucre et de 1 à 100 mg du polymère soluble dans l'eau sur la base d'une quantité équivalente à une teneur de 100 mg de cefditoren pivoxil.

6. Composition de dispersion solide selon l'une quelconque des revendications 1 à 4, qui contient de 5 à 100 mg de l'acide gras d'ester de sucre et de 1 à 50 mg du polymère soluble dans l'eau sur la base d'une quantité équivalente à une teneur de 100 mg de cefditoren pivoxil.

7. Composition de dispersion solide selon l'une quelconque des revendications 3 à 6, dans laquelle le polymère soluble dans l'eau pharmaceutiquement acceptable est un ou plusieurs polymères solubles dans l'eau choisis dans le groupe constitué de l'hydroxypropylméthylcellulose, de la méthylcellulose, de l'hydroxyéthylcellulose, de la polyvinylpyrrolidone et de l'hydroxypropylcellulose.

8. Composition de dispersion solide selon l'une quelconque des revendications 1 à 7, dans laquelle la durée de conservation de l'amorphie du cefditoren pivoxil est d'au moins 3 jours lorsqu'il est en suspension dans l'eau à une concentration de cefditoren pivoxil de 10 mg/ml.

9. Préparation pharmaceutique antibiotique comprenant la composition selon l'une quelconque des revendications 1 à 8 conjointement avec un additif pharmaceutiquement acceptable.

10. Composition liquide comprenant au moins 0,1 mg, de préférence au moins 5 mg, de l'acide gras d'ester de sucre sur la base d'une quantité équivalente à une teneur de 100 mg de cefditoren pivoxil, qui peut être obtenue en dissolvant ou en mettant en suspension une composition de dispersion solide selon l'une quelconque des revendications 1 à 8 ou une préparation pharmaceutique selon la revendication 9 dans un milieu.
